Europäisches Patentamt

European Patent Office

Office européen des brevets

(11) Publication number: **0 469 232 A1**

# EUROPEAN PATENT APPLICATION

(21) Application number: **91101769.7**

(22) Date of filing: **08.02.91**

(51) Int. Cl.⁵: **A61K 7/06**, A61K 7/09, A61K 7/13

(30) Priority: **03.08.90 JP 206217/90**
**09.11.90 JP 304377/90**

(43) Date of publication of application:
**05.02.92 Bulletin 92/06**

(84) Designated Contracting States:
**DE FR GB IT**

(71) Applicant: **KABUSHIKI KAISHA JAPAN HAPPY**
**3-16-12, Sanjo**

Suzuka-shi, Mie-ken(JP)

(72) Inventor: **Inoue, Tomio**
**1-14-3, Nakagawara**
**Yokkaichi-shi, Mie-ken(JP)**

(74) Representative: **Eisenführ, Speiser & Strasse**
**Balanstrasse 55**
**W-8000 München 90(DE)**

(54) Materials for hair cosmetics and hair cosmetics.

(57) The present invention provides a material for a hair cosmetic and a hair cosmetic capable of curing the hair damaged by cold permanent wave treatment, hair coloring treatment, etc. and improving the color, gloss, feel and combing smoothness of the hair. The material for hair cosmetic and the hair cosmetic contains at least one selected from among the group consisting of an extract obtained from corn grains by use of sulfurous acid water solution, water-soluble natural sugar, blood plasma or substitute blood plasma, shellac, keratin, carrageenan, a soluble cellulose, amylose, and an acrylic copolymer.

EP 0 469 232 A1

The present invention relates to materials for hair cosmetics containing various natural extract components and to hair cosmetics.

The hair is damaged by shampoo, permanent wave, hair coloring, ultraviolet of sunlight, salt content in perspiration. As a result, the color, gloss, feel and combing smoothness are degraded. In general, a liquid hair cosmetic consisting mainly of oil and one or more kinds of keratin (PPT) and proteins is used for the purpose of protection of the hair and hairdressing. The hair is coated with the hair cosmetic, and an oily coating layer is formed on the hair for protection and hairdressing. The liquid hair cosmetic consisting mainly of oil and one or more kinds of keratin and proteins, however, is unable to fully improve the color, gloss, feel and combing smoothness of the damaged hair. In addition, the oily coating layer for protection of the hair, which was formed by the hair cosmetic, is removed by shampoo; therefore, the coating layer is not satisfactory for the protection of the hair. Consequently, the effect for hairdressing is also lost shortly.

Permanent wave treatment and hair coloring treatment, which are typical causes for degradation of color, gloss, feel and combing smoothness of the hair, will now be explained in detail.

Permanent Wave Treatment

Permanent wave treatments include a cold two-bath type, a cold one-bath type, a heating two-bath type, etc. Of these, the cold two-bath type treatment is typical. In this treatment, a water solution (first liquid) is prepared, which is made to have an alkali-side pH by addition of a reducing agent, such as ammonium thioglycolate or monoethanolamine, and an alkali agent such as ammonia water. The first liquid is coated on the hair to cut hydrogen bonds, salt bonds and cystine bonds in the hair, thereby softening the hair. The softened hair is wound around curling rods. Then, after the first liquid is removed by hot water or acid rinse, a water solution (second liquid) containing soda bromate or bromates as an oxidizing agent is coated on the hair. Thus, the salt bonds are made once again, and the cystine bonds are also made once again by the action of the bromate. As the hair is drying, new hydrogen bonds are formed and the permanent wave treatment is completed.

In this treatment, however, the coating of the alkali water solution (first liquid) results in damage, peeling and change in color of the surface portion of the hair. In addition, the thioglycolate adversely affects the structure of the cortex of hair, and the strength and elasticity of hair, which are closely related to the structure of the cortex, decrease and the color, gloss, feel and combing smoothness of the hair are degraded. Furthermore, thinning of hair may occur. According to measured data, the moisture content in the hair prior to the permanent wave treatment was about 11 to 13 %, whereas the moisture content after the treatment fell to about 7 %.

Besides, the thioglycolate and ammonium remains in the hair after the permanent wave treatment, and a peculiar odor is emitted from the hair.

Hair Coloring Treatment

Coloring agents may employ vegetative, mineral or composite dyes. In general, a composite dye or an oxidation dye is used and coloring is effected by a two-liquid type process.

A mixture solution is prepared by mixing, immediately before hair coloring, ammonia water (first agent), to which an oxidation dye, an excipient and a surface-active agent serving as penetrating agent are added, and hydrogen peroxide water (second agent) serving as oxidizing agent. The prepared mixture solution is applied to the hair. The ammonia swells and softens the hair, and facilitates decomposition of the hydrogen peroxide, thereby generating oxygen. The oxygen effects hair coloring.

The mixture solution, however, contains an alkali agent and the oxidizing agent, which cause damage to the hair. It was found that after the hair coloring treatment, the color, gloss, feel and combing smoothness were degraded, and the color of hair faded shortly (about one month). If the hair coloring treatment is applied subsequently to permanent wave treatment, the hair would be doubly damaged. Thus, it is necessary to provide an interval of 7 to 10 days between the permanent wave treatment and hair coloring treatment, and it is not possible to continuously perform hair treatments (permanent wave treatment and coloring treatment).

The present invention has been made to solve the above problems, and its object is to provide materials for hair cosmetics and hair cosmetics capable of curing damaged hair and improving the color, gloss, feel and combing smoothness.

A material for hair cosmetics, according to the present invention, is a liquid containing at least one selected from among the group consisting of an extract obtained from corn grains by use of sulfurous acid water solution; water-soluble natural sugar; blood plasma or substitute blood plasma; carrageenan; shellac; a soluble cellulose; keratin; amylose; and an acrylic copolymer.

Two or more of these components may be combined, and the content ratio thereof and indispensable components are not strictly determined.

A hair cosmetic of this invention containing two or more components has a synergistic effect of these components and can better the color, gloss, feel and combing smoothness of the hair.

Regarding the above components, the extract from corn grains contains albumin and globulin as proteins and 16 kinds of amino acids, and provides the hair with nutrition and has excellent adhesion. The natural sugar is excellent in consolidation power, film-forming property, viscosity, gloss-providing property. The natural sugar also has a high adhesive force and a high water retention characteristic. The blood plasma is an animal protein, contains blood proteins of albumin and globulin and fibrinogen, and has a good adhesive force. Since the hair is containing the same elements of blood, these components have remarkable effects.

Specifically, in cold permanent wave treatment, cuticles of the hair, which were chemically/physically changed by thioglycolate and alkali agent at the time of cutting/reducing cystine bonds, are protected by the film-forming property, consolidation power and adhesive force of the natural sugar and blood plasma. Thus, damage or peeling of the cuticles is effectively prevented, damaged or peeled cuticles are cured, and the functions of the cuticles prior to the treatment using the reducing agent are maintained or recovered. The cuticles are cracked by thioglycolate and alkali agent, and the hair-matrix of the cortex flow out. The hair is compensated by blood plasma and natural sugar. The blood plasma is an animal protein and has good compatibility with human organism. Thus, the functions of the hair prior to the treatment are maintained or recovered. The natural resin, shellac, added to the treatment agent contains a great quantity of natural resin component (about 95 % or more) and a wax component (about 1.5 % to 2.5 %). The natural resin component and wax component form a coating film, which becomes artificial cuticles. The artificial cuticles protect the recovered hair, as if it was natural hair.

These functions are carried out effectively in cooperation with the alkali agent by which the hair is swelled. If the treatment agent of this invention is used, the hair is kept healthy even after subjected to cold permanent wave treatment.

On the other hand, the hair coloring agent of this invention, like the above permanent wave agent, contains an extract obtained from corngrains water-soluble natural sugar or blood plasma (or substitute blood plasma) and natural resin shellac and so on. Thus, like the above case, the damage to the hair due to hair coloring treatment can be prevented and the hair is kept healthy. In addition, the protection film is coated on the surface portion of the hair, thereby forming artificial cuticles of the hair. Thus, the color of the hair is stable for a long time, and the damage to the hair is negligible. Accordingly, the hair coloring treatment can be carried out immediately after the cold permanent wave treatment.

The treatment agent and hair coloring agent according to the present invention, to which "cyclodextrin" is added, can prevent the generation of peculiar odor of the alkali agent and thioglycolate.

A material for hair cosmetics, according to the present invention, is a liquid containing at least one selected from among the group consisting of an extract obtained from corn grains by use of sulfurous acid water solution; water-soluble natural sugar; blood plasma or substitute blood plasma; carrageenan; shellac; a soluble cellulose; keratin; amylose; and an acrylic copolymer.

The extract from corn grains is a compound of many soluble components. It was found that where the extract includes, as soluble components, at least inositol, choline and lactic acid, a "liquid" containing this extract improved the color, gloss, feel and combing smoothness of the hair.

In this context, the "liquid" means a water solution, emulsion, suspension, etc. containing the extract. The extract content in the "liquid" may be freely set, but typically it is about 10 to 60 % by weight.

For example, the components in the extract are:

(a) proteins such as water-soluble albumin and salt-soluble globulin;

(b) free amino acids such as aspartic acid, threonine, serine, glutamic acid, proline, glycine, alanine, valine, methionine, isoleucine, leucine, thyrosin, phenylalanine, histidine, lysine, and arginine;

(c) water-soluble vitamins such as thiamine, riboflavin, vitamin $B_6$, niacin, pantothenic acid, inositol, biotin, folic acid, and choline; and

(d) lactic acid.

The ratio of the components is generally as follows:

| | Extract from Corn Grains (%) |
|---|---|
| Water | 69.33 |
| Solid | 30.67 |
|     Proteins | (albumin) 7.0 |
| | (globulin) 10.0 |
|     Ash | 3.59 |
|     Salt | 4.05 |
|     Lactic Acid | 1.13 |
|     Balance | 4.9 |

The composition of the amino acids in the solid is as follows:

| Amubi Acids (%) | |
|---|---|
| Asp. | 0.038 |
| Thr. | 1.243 |
| Ser. | 0.586 |
| Glu. | 0.389 |
| Pro. | 1.284 |
| Gly. | 0.257 |
| Ala. | 1.653 |
| Cys. | 0 |
| Val. | 1.010 |
| Met. | 0.468 |
| I.Leu | 0.556 |
| Leu. | 2.649 |
| Tyr. | 0.276 |
| Phe. | 0.576 |
| His. | 2.420 |
| Lys. | 0.309 |
| Arg. | 0.185 |

The contents of the water-soluble vitamins in the solid are as follows:

| | |
|---|---|
| Thiamine | 1.05 mg/100g |
| Riboflavin | 0.39 mg/100g |
| Vitamin $B_6$ | 4.90 mg/100g |
| Niacin | 8.85 mg/100g |
| Pantothenic Acid | 9.08 mg/100g |
| Inositol | 1113 mg/100g |
| Biotin | 166.5 $\mu$g/100g |
| Folic Acid | 11 $\mu$g/100g |
| Choline | 0.44 % |

The extract from corn grains can be obtained by extraction with use of a specially prepared sulfurous acid water solution.

For example, after corn grains are subjected to extraction by use of a sulfurous acid water solution (e.g. concentration: about 0.1 %), an insoluble solid portion is separated from the solution. Water is evaporated from the solution containing the extract, and the resultant condensed water solution is brought into contact with an ion exchange substance (e.g. ion exchange resin) and is refined. Thus, the pH of the water solution

4

can be controlled to be 4 to 9.

The temperature in the extraction step is desirably 10 to 90°C, and more desirably 50°C. The time for extraction is desirably 10 to 100 hours, and more desirably 48 hours.

It is also possible to utilize the step of obtaining lactic acid by fermentation of corn grains. For example, fermentation is caused to occur in a mixture of lactic acid, corn grains and water, and an impurity is filtered. Then, water is evaporated from the mixture, and the mixture is condensed. Thus, the extract is obtained.

The water-soluble natural sugar may be a water-soluble monosaccharide, oligosaccharide, or polysaccharide, which is derived from a natural material (vegetable, animal). In this context, "water-soluble" means "water-soluble in a normal state" and does not mean "slightly water-soluble in high temperatures." The water-soluble natural sugar can be extracted from natural material (e.g. corn grains) by use of separating means. Saccharides may be used singly or in combination.

Specific examples of the water-soluble natural sugar are: glucose, maltose, dextran, Pruran (trade name, produced by HAYASHIBARA CO. LTD: a natural polysaccharide produced from "starch"), monosaccharide or polysaccharide derived from corn grains, alginic acid of heteropolysaccharide, pectic substance, guagum, chitin, xanthenegum, etc.

The chitin itself is water-insoluble; thus, it is made water-soluble for use (e.g. partially decomposed with use of an inorganic acid or formic acid).

Of the water-soluble natural saccharides, maltose is desirable.

Blood Plasma

As the blood plasma, not only the human blood plasma but also a substitute blood plasma produced artificially to achieve the same object as the human blood plasma can be employed. Typical substitute blood plasmas are a blood plasma obtained by partially decomposing dextran with use of a dilute acid and a polyvinylpyrrolidone.

Carrageenan is a mixture of a plurality of polysaccharides derived from sea weed (especially, red algae). The polysaccharides may have various molecular weights (e.g. 280,000 to 690,000). Typical repetition units of main chains of these polysaccharides are a bond of 3,6-anhydro-D-galactopyranose and D-galactopyranose-4-sulfuric acid, and a bond of two $\alpha$-D-galactopyranose-4-sulfuric acid. The carrageenan is extracted, along with a solvent, from sea weed, and the carrageenan is separated from the solvent. In the present invention, carrageenan obtained by any method can be used, irrespective of the kind of solvent (e.g. dilute alkali water solution, hot water) and the separating means.

In addition, in the present invention, polysaccharides of specific kinds (e.g. $x$-carrageenan, $\lambda$-carrageenan) separated from the carrageenan may be employed.

Although carrageenan can be used solely, if it is combined with blood plasma and polysaccharide, the water retention characteristic of the hair is enhanced. In this case, it is desirable that the mixing ratio of the carrageenan, blood plasma and polysaccharide be 0.1 to 10 % by weight: 0.1 to 10 % by weight: 0.5 to 80 % by weight (the total of carrageenan, blood plasma and polysaccharide is 100 % by weight).

Soluble Cellulose

A typical soluble cellulose is obtained by making the hydroxyl group of cellulose soluble by esterification or etherification.

Specific examples of soluble cellulose are methyl cellulose, hydroxypropylmethyl cellulose, and hydroxyethylmethyl cellulose. Of these, methyl cellulose is desirable.

Keratin

Keratin to be used may be any constituent protein contained in animal skin, hair, or eggshell.

The amount of added keratin is 0.1 to 40 % by weight (the total of carrageenan, cellulose, shellac, keratin, amylose and acrylic copolymer is 100 % by weight) and desirably 0.1 to 10 % by weight.

By virtue of the addition of keratin, the hair is coated with the keratin. Thus, after the hair is rinsed with water or shampooed, the improved color, gloss, feel and combing smoothness of the hair can be stably maintained.

Amylose

Amylose is a combination of several hundred to several thousand D-glucoses present in starch. In the normal state, amylose is not easily dissolved in water. In general, vegetative and animal glucoses may be used. A typical amylose is produced from corn grains.

The amount of added amylose is 0.5 to 50 % by weight (the total of carrageenan, cellulose, shellac, keratin, amylose and acrylic copolymer is 100 % by weight) and desirably 0.5 to 10 % by weight.

Amylose is added to a hair cosmetic, thereby giving a water retention property to the hair cosmetic.

Shellac

Shellac is composed mainly of a natural resin produced from an animal secretion. A wide range of products, which differ considerably in color and melting point, are called "shellac". Any type of shellac may be used. A coating film formed of natural fact (about 95 % or more in shellac) and wax (about 1.5 to 2.5 % in shellac) protects the hair, which has been cured by other components, and facilitates the curing. The coating film of natural fact and wax is not easily removed by shampoo. It is not removed even after 40 to 50 times of shampoo. Thus, the gloss and healthy condition of the hair can be stably maintained for a long time.

The amount of added shellac is 0.5 to 50 % by weight (the total of carrageenan, cellulose, shellac, keratin, amylose and acrylic copolymer is 100 % by weight) and desirably 0.5 to 10 % by weight.

By virtue of addition of shellac, the gloss of the hair is improved, and the improved state is kept for a long time. For example, where the hair cosmetic of the present invention, to which shellac is added, is applied to the hair, the gloss of the hair can be maintained even after 40 to 50 times of shampoo.

Acrylic Copolymer

A typical acrylic copolymer is a copolymer of acrylic monoglyceride and sodium acrylate.

The amount of added acrylic copolymer is 0.5 to 10 % by weight (the total of carrageenan, cellulose, shellac, keratin, amylose and acrylic copolymer is 100 % by weight) and desirably 0.5 to 2 % by weight.

A small amount of the copolymer of acrylic monoglyceride and sodium acrylate is added to the first liquid used in the cold two-bath type permanent wave treatment or the first agent and second agent used in the two-liquid type hair coloring treatment, thereby removing unpleasant odor of thioglycolate, ammonia or amino acid and enhancing the odor of perfume added to the cosmetic.

In the present invention, a liquid of one or more of the above-explained components is used as a hair cosmetic. The hair cosmetic is applied to the hair. Also, a liquid containing an extract may be added to the hair cosmetic.

In this context, "hair cosmetic" is typically a liquid-phase material applied to the hair in order to protect the hair, provide the hair with nutrition, or apply permanent wave treatment or hair coloring treatment. The modes of use of the hair cosmetic are not limited; for example, coating, spraying, etc.

The liquid containing the extract is typically a water solution or an emulsion. The water solution may be a condensed water solution obtained in the manufacturing process. The liquid, however, may be produced by dissolving the extract in refined water (e.g. water refined by various refining means) or in pure water.

Specific examples of the hair cosmetics are: (a) hair wash agents such as shampoo and hair rinse; (b) hair nutrition agents such as hair tonic and hair conditioner; (c) hair dressing agents such as pomade, hair oil, hair cream, hair liquid, set lotion, and hair spray; (d) permanent wave agent; and (e) hair coloring agents such as hair coloring substance, hair bleaching agent, color rinse, and color spray.

The permanent wave agent is a solution used for any one of the permanent wave treatments (e.g. cold two-bath type, cold one-bath type, heating two-bath type, etc.). For example, in the case of the cold two-bath type treatment, the permanent wave agent is the first and/or second liquids.

The mixture material in the present invention is a liquid material such as water solution or emulsion, containing 0.5 to 90 % by weight of an extract, 0.5 to 90 % by weight of a water-soluble natural sugar, and 0.5 to 90 % by weight of blood plasma (the total of the extract, water-soluble natural sugar and blood plasma is 100 % by weight). It is desirable that any one of the extract, natural sugar and blood plasma be contained in an amount of 1 to 2 % by weight and the others be the balance. The concentration of the total of these components is 0.5 to 90 % by weight in the liquid material and desirably 3 to 15 % by weight. Since this mixture material contains, in addition to the extract obtained with use of sulfurous acid water solution, the water-soluble natural sugar (capable of providing consolidation power, film-forming property, glossing property, adhesive force, and water retention characteristic) and the blood plasma (capable of providing stickiness), the damaged hair is cured, and the color, gloss, feel, combing smoothness and water retention characteristic (especially, water retention characteristic) are improved, and the improved state is maintained.

For example, when the hair was observed by a scanning electronic microscope with a magnifying power of 800, it was found that the damaged hair was recovered to the healthy condition.

By virtue of addition of cyclodextrin, the peculiar odor resulting from the component in the hair cosmetic remaining on the hair (e.g. odor of thioglycolate and ammonia after permanent wave treatment, and odor of ammonia after hair coloring treatment) can be removed.

The amount of cyclodextrin to be added may be small. Specifically, it is 0.1 to 10 % by weight and desirably 0.5 to 3 % by weight.

Specific cases wherein the mixture material according to the present invention is added to a permanent wave agent or a hair coloring agent will now be described in detail.

The mixture material of this invention is added to a permanent wave agent (e.g. first and second liquids in cold two-bath type treatment), thus preparing a permanent wave agent (e.g. emulsion) in which the concentration of the total of the respective components is 1 to 50 % by weight. Even after the hair is subjected to the permanent wave treatment, the recovery of the damaged hair is facilitated, and the color, gloss, feel and combing smoothness of the hair can be improved. In addition, hair coloring treatment can be carried out subsequently to the permanent wave treatment.

In the two-liquid type hair coloring treatment, the first liquid, second liquid, and mixture material are mixed just before coloring treatment, thereby preparing a mixture emulsion in which the concentration of the total of the respective components of the mixture material is 1 to 50 % by weight. The emulsion is applied to the hair.

The emulsion is prepared in the following manner. First, a solution (a) and a solution (b) are prepared. In the solution (a), a predetermined amount of soluble cellulose, shellac, keratin and amylose is dissolved in an alcohol solution or an alkali water solution. In the solution (b), the mixture additive of this invention is dissolved. Then, the solution (a), along with a surface-active agent, is mixed and stirred in the solution (b). Thus, the emulsion is prepared.

⟨Experimental Examples⟩

The present invention will now be described in greater detail, by referring to the following experimental examples. The present invention is not limited to these examples.

Example 1

Corn grains were put in a 0.1 % sulfurous acid water solution, and soluble components were extracted. The extraction was carried out for 48 hours at a temperature of 50°C of the water solution. After the extraction, a solid portion in the water solution was separated, and water was evaporated from the water solution. Thus, the solution was condensed and brought into contact with an ion exchange substance (e.g. ion exchange resin), whereby the solution was refined and the pH of the acid solution was controlled to be 7 to 9.

Shellac dissolved in a 2.5 % by weight of an alkali substance was added to the refined water containing the extraction thus obtained at a concentration of 3 % by weight. Thus, an emulsion hair cosmetic was prepared. The hair cosmetic was applied at room temperature to the hair after shampoo. Compared to the hair before application of hair cosmetic, the resultant hair had improved color, gloss and combing smoothness and was in the healthy condition.

Thereafter, the hair was subjected to shampoo every day, and the health condition, color, gloss and combing smoothness were examined. It was found that the healthy condition of the hair was maintained even after 45 days in which the hair was shampooed every day.

Example 2

A refined water solution in which 10 % by weight of water-soluble natural sugar was dissolved was mixed with "alkali-dissolved shellac" in an amount corresponding to 3 % by weight of the refined water solution. Thus, an emulsion-phase hair treatment agent was prepared. The treatment agent was applied at room temperature to the hair after it was shampooed. Compared to the hair before application of treatment, the resultant hair had the color, gloss, combing smoothness and hair diameter which were recovered substantially to the healthy condition. Desirable treatment was carried out. Then, the hair was subjected to normal shampoo every day, and the condition of the hair was observed. The good condition of the hair, which did not require the next use of treatment agent, was stably maintained 45 days after the application of the treatment agent.

Example 3

A stock solution was prepared by adding glucose, blood plasma and natural resin shellac to the main component consisting of thioglycolate and alkali agent. The stock solution was mixed with vegetative oil, an emulsifier and water, thus preparing an emulsion. The solution having a pH of 8 and containing thioglycolate, glucose, blood plasma and shellac at a ratio of 7 % (by weight): 5 %: 5 %: and 2.5 % was used as a first liquid of a reducing agent. Using the first liquid and the above-mentioned second liquid of oxidizing agent, the hair was subjected to cold permanent wave treatment.

The peeling and damage of surface portion of the treated hair was negligible, the moisture content of the hair was 13 %, and thinning of hair was not observed. The hair remained in the good condition in which the hair was protected by cuticles. Even after the treatment, the hair had good color, gloss and combing smoothness, and the damage to the hair in conventional cold permanent wave treatment was not found at all.

Example 4

A water solution containing 50 % by weight of an extract from corn grains, 6 % by weight of dextran (partially decomposed by dilute acid), 12 % by weight of glucose, and 6 % by weight of Pruran. Then, 6 mℓ of this water solution was added to 800 mℓ of the first liquid used in permanent wave treatment (cold two-bath type). Using the first liquid, the permanent wave treatment was applied to the damaged hair having a moisture content of 7 % or less. The hair, after permanent wave treatment, was examined and it was found that the hair had a moisture content of 11 to 13 %.

The hair, before permanent wave treatment, was rough and had bad combing smoothness. After the permanent wave treatment, the hair had good combing smoothness as well as good gloss.

The hair, before the permanent wave treatment, had a fading color, compared to the healthy hair. After the permanent wave treatment, the brightness of the hair (measured on the basis of a color chart for hair coloring) increased by about 0.5 to 1 tone. The surface of the hair was illuminated and observed, and it was found that the hair was in the healthy condition, like the child's hair.

Example 5

A hair coloring agent was prepared by combining a first agent and a second agent. The first agent was an emulsion made by adding Pruran (or a natural polysaccharide), blood plasma and natural resin shellac to the main component consisting of an oxidizing coloring agent and an alkali agent (the ratio of Pruran, blood plasma and shellac was 5 % (by weight): 5 %: 2.5 %). The second agent was composed of an oxidizing agent. Immediately after the hair was subjected to the cold permanent wave treatment in Example 1, it was subjected to hair coloring treatment.

The colored hair was substantially unchanged from the time just after the hair was subjected to the treatment of Example 1. The resultant hair had a healthy condition wherein the hair was protected by cuticles. There was no damage to the hair which occurred in conventional hair coloring treatment. In addition, the color did not fade for a long time (three months) and the stability of the color of hair was confirmed.

Example 6

A water solution was prepared, which contained 50 % by weight of an extract obtained from corn grains by use of a 0.1 % sulfurous acid water solution, 6 % by weight of dextran, 12 % by weight of maltose and 6 % by weight of Pruran. 6 mℓ of this water solution was mixed in 60 g of a first agent used in a two-liquid type hair coloring treatment using an oxidizing coloring agent. A mixture solution prepared by mixing the first agent (containing 6 mℓ of the water solution) and hydrogen peroxide (second agent) just before hair coloring treatment was applied to the hair. Compared to the hair before the coloring treatment, the resultant colored hair had much better gloss and combing smoothness, and the color of hair has an increased depth. Specifically, the colored hair had excellent feel, gloss and coming smoothness.

Thereafter, the hair was shampooed every other day. In the case of conventional hair coloring treatment using a mixture solution consisting of only the first and second agents, color fading was observed about 20 to 35 after the treatment; by contrast, according to this treatment, color fading was not observed even after 60 days.

Example 7

100 mℓ of water solution was prepared, which contains 30 mℓ of condensed water solution containing 10 g of Pruran and an extract from corn grains at a concentration of 50 % by weight; 30 mℓ of alkali water solution containing 15 g of glucose, 10 g of maltose and shellac at a concentration of 25 % by weight; carrageenan at a concentration of 0.5 % by weight; and refined water. 30 mℓ of this water solution was applied to the shampooed hair. The hair was covered with a hair steamer at a temperature of 45°C for 15 to 20 minutes. Then, the hair was washed and set.

Compared to the hair to which such water solution was not applied after shampoo, the resultant hair had much better gloss and combing smoothness. In particular, the gloss of hair was beautiful when the hair was illuminated by sunlight.

Split hairs of about 3 mm, which were observed at end portions of the hair before the treatment, were eliminated, and the elasticity of hair was recovered to the healthy condition.

Even after 30 days from the setting of the hair, the feel of the hair was excellent and the gloss and combing smoothness was unchanged from the time just after the setting.

Example 8

300 mℓ of mixture water solution was prepared, which consists of 100 mℓ of water solution containing an extraction from corn grains at a concentration of 50 % by weight; 100 mℓ of water solution containing methyl cellulose at a concentration of 10 % by weight and keratin at a concentration of 15 % by weight; 50 mℓ of water solution containing maltose at a concentration of 70 % by weight; and 50 mℓ of water solution containing dextran (partially decomposed with use of dilute acid) at a concentration of 50 % by weight.

30 mℓ of this mixture water solution was substituted for the water solution used in Example 7, and was used in the same manner as in Example 7. As a result, the same results as mentioned above were obtained.

**Claims**

1. A material for a hair cosmetic containing at least one selected from among the group consisting of an extract obtained from corn grains by use of sulfurous acid water solution; water-soluble natural sugar; blood plasma or substitute blood plasma; shellac; keratin; carrageenan; a soluble cellulose; amylose; and an acrylic copolymer.

2. The material for a hair cosmetic according to claim 1, characterized in that said extract obtained from corn grains by use of sulfurous acid water solution includes inositol, choline and lactic acid.

3. A liquid mixture material for a hair cosmetic containing 0.5 to 90 % by weight of an extract obtained from corn grains by use of sulfurous acid water solution, 0.5 to 90 % by weight of water-soluble natural sugar, and 0.5 to 90 % by weight of blood plasma.

4. A liquid material for a hair cosmetic containing 10 to 90 % by weight of a total of a mixture material of an extract obtained from corn grains by use of sulfurous acid water solution, water-soluble natural sugar, and blood plasma; and 90 to 10 % by weight of at least one selected from among the group consisting of carrageenan, shellac, a soluble cellulose, keratin, amylose, and an acrylic copolymer.

5. A cold permanent wave treatment agent characterized in that a solution, in which the material according to claim 1 is added to a main component consisting of a main agent of thioglycolate or a salt thereof and an alkali agent, is used as a reducing agent.

6. A hair coloring agent comprising a first agent, characterized in that the material according to claim 1 is added to a main component consisting of an oxidizing coloring agent and an alkali agent, and a second agent consisting mainly of an oxidizing agent.

7. The hair cosmetic according to claim 4, characterized in that said hair cosmetic is a hair treatment agent.

8. The hair cosmetic according to claim 4, characterized in that said hair cosmetic is a reducing agent for cold permanent wave treatment.

9.  The hair cosmetic according to claim 4, characterized in that said hair cosmetic is a hair coloring agent.

10. The material according to claim 1 or the hair cosmetic according to claim 4, characterized by further comprising cyclodextrin.

11. The hair cosmetic according to claim 10, characterized in that the amount of said cyclodextrin to be added is 0.1 to 10 % by weight and desirably 0.5 to 3 % by weight.

| | DOCUMENTS CONSIDERED TO BE RELEVANT | | EP 91101769.7 | |
|---|---|---|---|---|
| **Category** | Citation of document with indication, where appropriate, of relevant passages | Relevant to claim | CLASSIFICATION OF THE APPLICATION (Int. Cl.5) | |
| X | PATENT ABSTRACTS OF JAPAN, unexamined applications, C field, vol. 13, no. 137, April 5, 1989 THE PATENT OFFICE JAPANESE GOVERNMENT page 29 C 582 * Kokai-no. 63-301 809 (KAO CORPORATION) * | 1 | A 61 K 7/06 A 61 K 7/09 A 61 K 7/13 | |
| A | -- | 4,7 | | |
| X | PATENT ABSTRACTS OF JAPAN, unexamined applications, C field, vol. 9, no. 1, January 5, 1985 THE PATENT OFFICE JAPANESE GOVERNMENT page 119 C 259 * Kokai-no. 59-157 010 (KAO SEKKEN K.K.) * | 1 | | |
| A | -- | 4,7 | TECHNICAL FIELDS SEARCHED (Int. Cl.5) | |
| X | PATENT ABSTRACTS OF JAPAN, unexamined applications, C field, vol. 13, no. 227, May 25, 1989 THE PATENT OFFICE JAPANESE GOVERNMENT page 153 C 600 * Kokai-no. 1-40 415 (SAN EI CHEM IND. LTD.) * | 1 | A 61 K 7/00 | |
| A | -- | 4,7 | | |
| X | US - A - 4 374 825 (R. E. BOLICH et al.) * Claims; column 2, line 61 - column 3, line 11 * | 1 | | |
| A | -- | 4,7 | | |
| X | EP - A2 - 0 279 016 | 1,6 | | |

The present search report has been drawn up for all claims

| Place of search | Date of completion of the search | Examiner |
|---|---|---|
| VIENNA | 22-10-1991 | IRMLER |

EPO FORM 1503 03.82 (P0401)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| | (HOYU CO) <br>     * Claims; page 3, lines <br>       25-30 * | | |
| A | | 4,7,9, <br> 10,11 | |
| X | US - A - 4 685 913 <br> (L. SCHIEFERSTEIN) <br>     * Claims * | 1,6 | |
| A | | 4,9 | |
| X | EP - A1 - 0 005 807 <br> (HENKEL) <br>     * Claims; page 2, lines <br>       20-28 * | 1 | |
| A | | 3,4,6 | |
| X | HANDBUCH DER KOSMETIKA UND <br> RIECHSTOFFE, vol. 1, 2. ed., <br> 1969 <br> H. JANISTYN "Die kosmetischen <br> Grundstoffe" Dr. Alfred Hüthig <br> Verlag, Heidelberg <br> pages 889,890 | 1 | TECHNICAL FIELDS <br> SEARCHED (Int. Cl.5) |
| A | | 4 | |
| A | US - A - 4 180 561 <br> (W. L. VINSON) <br>     * Claims * | 1-5,7 | |
| A | DE - A1 - 3 130 894 <br> (M. KRIWET) <br>     * Totality * | 1-4,7 | |
| A | EP - A2 - 0 180 968 <br> (EXOVIR) <br>     * Claims * | 1-4 | |

The present search report has been drawn up for all claims

| Place of search | Date of completion of the search | Examiner |
|---|---|---|
| VIENNA | 22-10-1991 | IRMLER |

EPO FORM 1503 03.82 (P0401)